# EUROPÄISCHE PATENTANMELDUNG

(11) **EP 0 920 834 A1**
(43) Veröffentlichungstag der Anmeldung: **09.06.1999**
(21) Anmeldenummer: 98122849.7
(22) Anmeldetag: 02.12.1998
(51) Int. Cl.: A61B 13/00

(54) **Mundspatel**

(30) Priorität: 02.12.1997 DE 19753343
(71) Anmelder: Benediktus Kräuterlabor Strathausen GmbH, 84028 Landshut (DE)
(72) Erfinder: Strathausen, Gerold, 84079 Bruckberg (DE)
(74) Vertreter: Pausch, Thomas, Dipl.-Phys.

(57) **Zusammenfassung**

Die Erfindung bezieht sich auf ein Mundspatel aus einem länglichen, im wesentlichen abgeflachten Grundkörper (2) mit einem Griffende (3) und einem gegenüberliegenden, mit einer Süssmasse versehenen Druckende (4). Die Süssmasse (5) ist durch ein das Druckende (4) wenigstens bereichsweise umfassendes und das Druckende (4) verankerndes Formteil, insbesondere Spritzgußformteil gebildet, welches ein den Rand des Druckendes übergreifendes Halteelement (5a) zur Festlegung der Süssmasse (5) mit dem Grundkörper (2) besitzt. Die Süssmasse (5) besteht somit aus dem Halteelement und einem die weitere Teilfläche des Druckendes vollständig bedeckenden Geschmackselement (5b), wobei der längliche Grundkörper (2) in dem bei Zusammenfügen der beiden Elemente sich ergebenden Hohlraum (10) formschlüssig eingesetzt ist.

## Beschreibung

Die Erfindung bezieht sich auf ein Mundspatel aus einem länglichen, im wesentlichen abgeflachten Grundkörper mit einem Griffende und einem gegenüberliegenden, mit einer Süssmasse versehenen Druckende, und ein Verfahren zu seiner Herstellung.

Ein solcher Mundspatel zur Untersuchung der Mundhöhle insbesondere von kleinen Patienten in der kinderärztlichen Praxis ist beispielsweise aus der DE-G 71 22 076 bekannt. Bei diesem und bei einigen auf dem Markt erhältlichen Mundspatel ist die am Druckende angebrachte Süssmasse nur unzureichend mit dem Grundkörper fixiert, wobei die Gefahr besteht, dass sich die Süssmasse beim Lutschen plötzlich ablöst und in den Rachen des Kindes gelangt, was zu Erstickungsanfällen insbesondere bei Kleinkindern führen kann.

Der Erfindung liegt daher die Aufgabe zugrunde, einen Mundspatel der gattungsgemäßen Art dahingehend zu verbessern, dass Gefährdungen des Patienten ausgeschlossen sind.

Diese Aufgabe wird vorrichtungsgemäß durch ein Mundspatel nach Anspruch 1 und verfahrensgemäß durch das im Anspruch 7 angegebene Verfahren gelöst.

Die Erfindung schlägt ein Mundspatel vor, bei dem die Süssmasse durch ein das Druckende wenigstens bereichsweise umfassendes und das Druckende verankerndes Formteil, insbesondere Spritzgußformteil gebildet ist.

Bei einer bevorzugten konstruktiven Ausgestaltung besitzt das Formteil ein den Rand des Druckendes übergreifendes Halteelement zur Festlegung der Süssmasse mit dem Grundkörper. Zweckmässig ist das Halteelement dem Rand des Druckendes folgend wulstförmig ausgebildet, wobei der den Rand des Druckendes umfassende Wulst einen Streifen von wenigstens etwa 1 bis 2 mm auf der Teilfläche des Druckendes bedeckt.

Bei einer bevorzugten Ausführung ist ferner vorgesehen, dass die Süssmasse zweiteilig aus dem Halteelement und einem die weitere Teilfläche des Druckendes vollständig bedeckenden Geschmackselement besteht, und der längliche Grundkörper in dem bei Zusammenfügen der beiden Elemente sich ergebenden Hohlraum formschlüssig eingesetzt ist.

Von Vorteil enthält die Süssmasse des erfindungsgemässen Spatels nur reinste Naturstoffe und ist zuckerfrei.

Das erfindungsgemäße Verfahren zeichnet sich dadurch aus, dass die Süssmasse zweiteilig aus dem Halteelement und einem die weitere Teilfläche des Druckendes vollständig bedeckenden Geschmackselement gefertigt, und unter Einschluß des Druckendes des länglichen Grundkörpers zusammengefügt werden.

Bevorzugte Weiterbildungen der Erfindung ergeben sich aus den Unteransprüchen.

Weitere Merkmale, Vorteile und Zweckmäßigkeiten der Erfindung ergeben sich aus der Beschreibung eines Ausführungsbeispieles anhand der Zeichnung. Es zeigen:

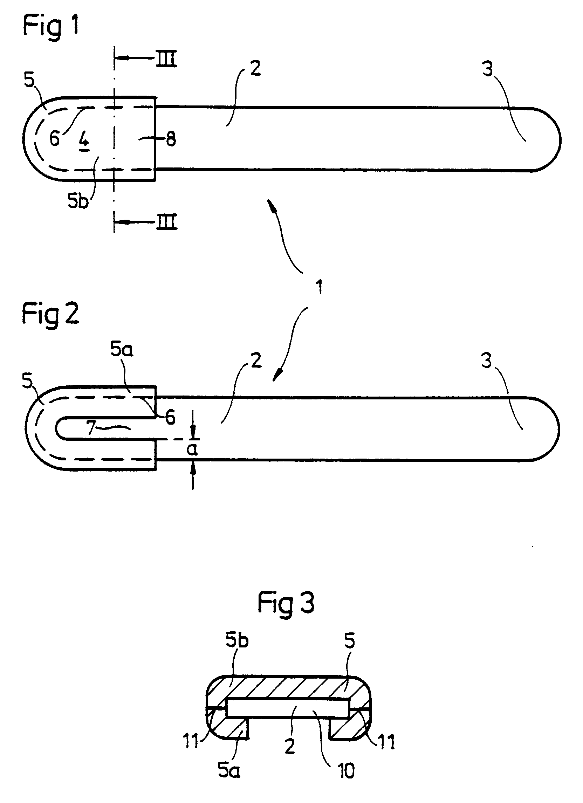

Figur 1 die Draufsicht eines Mundspatels gemäß der Erfindung,
Figur 2 die Unteransicht des Mundspatels, und
Figur 3 eine Schnittansicht des Mundspatels.

Der in den Figuren gezeigte Mundspatel 1 besteht aus einem länglichen, abgeflachten, zungenförmigen Grundkörper 2 aus Holz oder Kunststoff mit einem Griffende 3 und einem gegenüberliegenden Druckende 4. Bei der Untersuchung eines Patienten wird der Mundspatel 1 am Griffende 3 erfasst, so dass mit dem Druckende 4 die Zunge des Patienten niedergedrückt werden kann.

Gemäß der Erfindung ist das Druckende 4 mit einer einen Geschmacksstoff enthaltenden Süssmasse 5 versehen, der von Vorteil lediglich reinste Naturstoffe (beispielsweise reine Fruchtmittel als Farbstoff) enthält und zuckerfrei ist. Die Süssmasse 5 ist durch ein das abgerundete Druckende wenigstens bereichsweise umfassendes und das Druckende 4 verankerndes Formteil, insbesondere Spritzgussformteil ausgebildet. Das Formteil umfasst hierbei ein den Rand 6 des Druckendes 4 übergreifendes Halteelement 5a zur Festlegung der Süssmasse mit dem Grundkörper 2. Hierbei ist das Halteelement 5a dem abgerundeten Rand 6 des Druckendes 4 folgend wulstförmig ausgebildet, wobei der Wulst auf der Teilfläche 7 einen Streifen mit einer Breite a von etwa 1 bis 2 mm bedeckt. Die Süssmasse 5 ist zweiteilig und besteht neben dem Halteelement 5a aus einem die weitere Teilfläche 8 des Druckendes 4 vollständig bedeckenden Geschmackselement 5b, wobei die beiden Elemente 5a und 5b dergestalt zusammengesetzt sind, dass der längliche Grundkörper 2 in dem bei Zusammenfügen der beiden Elemente sich ergebenden Hohlraum 10 formschlüssig eingesetzt ist. Damit ergibt sich eine sichere Befestigung der Süssmasse 5 mit dem Grundkörper 2, wodurch jederzeit ein Loslösen der Süssmasse 5 von dem Spatel wirksam verhindert ist. Eine Gefährdung des Kindes durch Verschlucken der Süssmasse ist damit ausgeschlossen.

Die Herstellung des erfindungsgemäßen Mundspatels 1 erfolgt durch eine Spritzgusstechnik. Hierbei werden die beiden Bestandteile der Süssmasse 5, nämlich das Halteelement 5a und das Geschmackselement 5b in einer geeignet gestalteten Form einer Spritzgussapparatur in noch zähfließender oder wenigstens annähernd fluider Konsistenz der Süssmasse geformt, und unter Einschluss des Druckendes 4 des länglichen Grundkörpers 2 unter Pressdruck zusammengefügt. An den Verbindungsstellen 11 gegebenenfalls übrigbleibende Grate werden entfernt.

## Patentansprüche

1. Mundspatel aus einem länglichen, im wesentlichen abgeflachten Grundkörper (2) mit einem Griffende (3) und einem gegenüberliegenden, mit einer Süssmasse versehenen Druckende (4),
dadurch gekennzeichnet, dass
die Süssmasse (5) durch ein das Druckende (4) wenigstens bereichsweise umfassendes und das Druckende (4) verankerndes Formteil, insbesondere Spritzgußformteil gebildet ist.

2. Mundspatel nach Anspruch 1, dadurch gekennzeichnet, dass das Formteil ein den Rand des Druckendes übergreifendes Halteelement (5a) zur Festlegung der Süssmasse (5) mit dem Grundkörper (2) besitzt.

3. Mundspatel nach Anspruch 2, dadurch gekennzeichnet, dass das Halteelement (5a) dem Rand des Druckendes (4) folgend wulstförmig ausgebildet ist.

4. Mundspatel nach einem der vorhergehenden Ansprüche, dadurch gekennzeichnet, dass der den Rand (6) des Druckendes (4) umfassende Wulst einen Streifen mit einer Breite a von wenigstens etwa 1 bis 2 mm auf der Teilflache (7) des Druckendes (4) bedeckt.

5. Mundspatel nach einem der vorhergehenden Ansprüche, dadurch gekennzeichnet, dass die Süssmasse (5) zweiteilig aus dem Halteelement und einem die weitere Teilfläche des Druckendes vollständig bedeckenden Geschmackselement (5b) besteht, und der längliche Grundkörper (2) in dem bei Zusammenfügen der beiden Elemente sich ergebenden Hohlraum (10) formschlüssig eingesetzt ist.

6. Mundspatel nach einem der vorhergehenden Ansprüche, dadurch gekennzeichnet, dass die Süssmasse (5) nur reinste Naturstoffe enthält und zuckerfrei ist.

7. Verfahren zur Herstellung eines Mundspatel aus einem länglichen, im wesentlichen abgeflachten Grundkörper (2) mit einem Griffende (3) und einem gegenüberliegenden, mit einer Süssmasse (5) versehenen Druckende (4),
dadurch gekennzeichnet, dass
die Süssmasse (5) durch ein das Druckende (4) wenigstens bereichsweise umfassendes und das Druckende (4) verankerndes Formteil, insbesondere Spritzgußformteil gebildet wird.

8. Verfahren nach Anspruch 7, dadurch gekennzeichnet, dass das Formteil als ein den Rand des Druckendes übergreifendes Halteelement zur Festlegung der Süssmasse (5) mit dem Grundkörper (2) gefertigt wird.

9. Verfahren nach Anspruch 8, dadurch gekennzeichnet, dass das Halteelement dem Rand des Druckendes folgend wulstförmig ausgebildet wird.

10. Verfahren nach einem der Ansprüche 7 bis 9, dadurch gekennzeichnet, dass der den Rand des Druckendes umfassende Wulst einen Streifen von wenigstens etwa 1 bis 2 mm auf der Teilfläche des Druckendes bedeckt.

11. Verfahren nach einem der Ansprüche 7 bis 10, dadurch gekennzeichnet, dass das die Süssmasse (5) zweiteilig aus dem Halteelement und einem die weitere Teilfläche des Druckendes vollständig bedeckenden Geschmackselement gefertigt, und unter Einschluß des Druckendes des länglichen Grundkörper (2)s zusammengefügt werden.

12. Verfahren nach einem der vorhergehenden Ansprüche 7 bis 11, dadurch gekennzeichnet, dass die Süssmasse (5) nur reinste Naturstoffe enthält und zuckerfrei ist.
